Europäisches Patentamt

European Patent Office

Office européen des brevets

(19)

(11) Veröffentlichungsnummer: **0 264 065**
**B1**

(12) **EUROPÄISCHE PATENTSCHRIFT**

(45) Veröffentlichungstag der Patentschrift:
26.09.90

(51) Int. Cl.⁵: **C07C 21/06, C07C 17/34,**
**B01D 3/06**

(21) Anmeldenummer: 87114696.5

(22) Anmeldetag: 08.10.87

(54) Verfahren zur Herstellung von Vinylchlorid durch thermische Spaltung von 1,2-Dichlorethan.

(30) Priorität: 10.10.86 DE 3634550
10.02.87 DE 3704028

(43) Veröffentlichungstag der Anmeldung:
20.04.88 Patentblatt 88/16

(45) Bekanntmachung des Hinweises auf die Patenterteilung:
26.09.90 Patentblatt 90/39

(84) Benannte Vertragsstaaten:
BE DE ES FR GB GR IT NL SE

(56) Entgegenhaltungen:
EP-A- 0 180 925
EP-A- 0 225 617

(73) Patentinhaber: HOECHST AKTIENGESELLSCHAFT,
Postfach 80 03 20, D-6230 Frankfurt am Main 80(DE)
Patentinhaber: Uhde GmbH,
Friedrich-Uhde-Strasse 15 Postfach 262,
D-4600 Dortmund 1(DE)

(72) Erfinder: Link, Gerhard, Aubachstrasse 55a,
D-6500 Mainz(DE)
Erfinder: Fröhlich, Walter, Dr., Kampenwanndstrasse 11,
D-8269 Burgkirchen(DE)
Erfinder: Krumböck, Reinhard, Lohnerstrasse 40,
D-8269 Burgkirchen(DE)
Erfinder: Prantl, Georg, Pergerstrasse 16,
D-8263 Burghausen(DE)
Erfinder: Schaffelhofer, Iwo, Dr., Kettelerstrasse 2,
D-8263 Burghausen(DE)

## Beschreibung

Die Erfindung betrifft ein Verfahren zur Herstellung von Vinylchlorid gemäß Anspruch 1 und eine Vorrichtung zur Durchführung des Verfahrens gemäß Anspruch 14.

Die unvollständige, thermische Spaltung von 1,2-Dichlorethan, bei Drucken von 1 bis 4 MPa und Temperaturen von 450 bis 550 °C, zur Gewinnung von Vinylchlorid wird seit vielen Jahren großtechnisch betrieben. Ein Problem hierbei sind die sich bei den hohen Temperaturen bildenden Nebenprodukte, die unter anderem zur Bildung von Koks und Verstopfungen in den Rohren des Pyrolyse-Ofens führen können. Es ist daher üblich, diese Nebenprodukte vom nicht-umgesetzten 1,2-Dichlorethan, bevor es in den Prozeß zurückgeführt wird, destillativ abzutrennen. In diesem gereinigten 1,2-Dichlorethan bilden sich jedoch während der Aufheizung und besonders während der Verdampfung vor der Spaltung erneut unerwünschte Nebenprodukte, die während der Spaltung zu Schwierigkeiten durch Anbackungen an den Spaltrohren führen.

Zur teilweisen Abtrennung dieser Nebenprodukte ist es aus DE-OS 23 13 037 bekannt, 1,2-Dichlorethan im oberen Teil des Pyrolyse-Ofens bei einer Temperatur von 200 bis 250 °C und einem Druck von 20 bis 35 atü (ca. 2 bis 3,5 MPa) teilweise zu verdampfen, aus dem Gemisch von dampfförmigem und flüssigem 1,2-Dichlorethan die flüssigen Anteile in einem Abscheider abzutrennen und nach Filtration gegebenenfalls unter Zumischung von frischem 1,2-Dichlorethan in den oberen Teil des Pyrolyse-Ofens zurückzuführen, während die über Kopf des Abscheiders abziehenden 1,2-Dichlorethan-Dämpfe in den unteren Teil des Pyrolyse-Ofens eingeleitet und dort gespalten werden. Die partielle Verdampfung des 1,2-Dichlorethans kann in einem Apparat erfolgen, der getrennt vom Pyrolyse-Ofen mit Brennstoff beheizt wird. Zur Förderung des flüssigen 1,2-Dichlorethans wird eine Pumpe verwendet.

Dieses Verfahren ist durch Pumpe und Filter apparativ aufwendig und störanfällig. Es gestattet lediglich, die in den Rauchgasen des Pyrolyse-Ofens, jedoch nicht die in den Spaltgasen enthaltene Wärme zu nutzen. Es reinigt das erhitzte 1,2-Dichlorethan nur von festen (Koks) Partikeln, jedoch nicht von ebenso unerwünschten, flüssigen Verunreinigungen, die bei der späteren Spaltung erfahrungsgemäß Koks bilden. In der Praxis wird ein Spalt-Umsatz von höchstens 54 % erreicht.

Zur Verwendung der Wärme, die in den vinylchloridhaltigen Spaltgasen enthalten ist, ist es aus DE-OS 29 13 030 bekannt, diese Gase in einem Wärmetauscher zu kühlen, der mantelseitig von flüssigem 1,2-Dichlorethan als Kühlmittel durchströmt wird. Letzteres wird nach Wärmeaufnahme gasförmig der Spaltzone zugeführt. Eine Reinigung des 1,2-Dichlorethans während oder nach der Erwärmung ist nicht vorgesehen, es kann auch kein höherer Umsatz als nach dem Verfahren der DE-OS 23 13 037 erzielt werden. Ein weiterer Nachteil ist die starre Kopplung des energieliefernden Spaltgases, mit dem zu erwärmenden 1,2-Dichlorethan-Strom, wobei Schwankungen, die bei einer Produktion immer vorkommen, nicht ausgeglichen werden können.

Es wurde nun ein Verfahren gefunden, das es ermöglicht, die in den Spaltgasen enthaltene Wärme auszunützen, das erwärmte 1,2-Dichlorethan von festen und höher siedenden, flüssigen Verunreinigungen zu reinigen und Produktionsschwankungen flexibel auszugleichen, wobei ein höherer Spaltumsatz bei vergleichbarer Laufzeit als mit den Verfahren nach dem Stand der Technik erzielt wird.

Das neue Verfahren zur Herstellung von Vinylchlorid durch thermische Abspaltung von Chlorwasserstoff aus 1,2-Dichlorethan in einem Spaltofen, wobei flüssiges 1,2-Dichlorethan mit dem heißen, Vinylchorid enthaltenden Gas, das den Spaltofen verläßt, indirekt erwärmt, verdampft und das gasförmige 1,2-Dichlorethan in den Spaltofen eingeführt wird, ist dadurch gekennzeichnet, daß das 1,2-Dichlorethan in einem ersten Behälter mit dem Vinylchlorid enthaltenden Gas bis zum Siedepunkt erwärmt wird und von dort in einen zweiten darüberliegenden Behälter aufsteigt, in dem es ohne weitere Erwärmung unter geringerem Druck als in dem ersten Behälter teilweise verdampft, wobei das verdampfte 1,2-Dichlorethan in den Spaltofen eingespeist und das nicht verdampfte 1,2-Dichlorethan in den ersten Behälter zurückgeführt wird.

Das verdampfte 1,2-Dichlorethan wird durch Zuführung von frischem, flüssigem 1,2-Dichlorethan ersetzt, wobei die Standhöhe des flüssigen 1,2-Dichlorethans im zweiten Behälter vorteilhaft so gehalten wird, daß eine große Flüssigkeitsoberfläche zur Verfügung steht. In einer bevorzugten Ausführungsform des neuen Verfahrens wird das frische, flüssige 1,2-Dichlorethan in den zweiten Behälter eingespeist, wobei die Temperatur vorher mit Temperiermitteln, wie zum Beispiel Wasser, Wasserdampf, 1,2-Dichlorethan enthaltenden Flüssigkeiten, die an anderer Stelle des Prozesses wieder eingesetzt werden, oder Öl, geregelt wird. Hierbei wird zweckmäßig die Standhöhe der Flüssigkeit im zweiten Behälter als Regelgröße verwendet.

Die Menge des verdampften 1,2-Dichlorethans kann in weiten Grenzen schwanken. Vorteilhaft werden je Quadratmeter Oberfläche, der im zweiten Behälter als ruhend angenommen Flüssigkeit 1 000 bis 10 000 kg pro Stunde und insbesondere 2 000 bis 5 000 kg pro Stunde 1,2-Dichlorethan verdampft. Die Oberfläche, der als ruhend angenommen Flüssigkeit kann leicht aus den Abmessungen des zweiten Behälters,unter Berücksichtigung der Standhöhe der Flüssigkeit,ermittelt werden. Tatsächlich ist die Oberfläche während der Durchführung des Verfahrens in ständiger Bewegung, wodurch sie etwas größer als die als ruhend angenommene Oberfläche ist. Die Bestimmung der schnell wechselnden tatsächlichen Oberfläche ist jedoch äußerst schwierig, wenn nicht gar unmöglich.

Die Temperatur, mit der das frische, flüssige 1,2-Dichlorethan den beiden Behältern, die zur Verdampfung verwendet werden, zugeführt wird, kann in weiten Grenzen schwanken, sie ist nach unten durch die in den Spaltgasen enthaltene Wärme begrenzt, die obere Grenze kann wenige Grade unter der Temperatur liegen, bei der das 1,2-Dichlorethan aus dem zweiten Behälter verdampft. Zweckmäßig wird das 1,2-Dichlorethan bereits vorgewärmt,für das erfindungsgemäße Verfahren verwendet. Vorteilhaft wird das frische, flüssige 1,2-Dichlorethan mit einer Temperatur von 150 bis 220 °C insbesondere von 170 bis 210 °C dem zweiten Behälter zugeführt, wobei diese Temperatur so gewählt wird, daß sie mindestens 20 °C unter der Temperatur liegt, mit der das 1,2-Dichlorethan den zweiten Behälter gasförmig verläßt. Der Druck des 1,2-Dichlorethans soll während der Vorwärmung bis zur Einführung in den zweiten Behälter so hoch liegen, daß ein vorzeitiges Sieden der zugeführten Flüssigkeit vermieden wird.

Zur Vorwärmung des flüssigen, frischen 1,2-Dichlorethans, sind verschiedene Methoden geeignet, beispielsweise kann sie mit Wasserdampf, erhitzten hochsiedenden Flüssigkeiten, beispielsweise Mineralöl oder geschmolzenes Diphenyl, mit den heißen Verbrennungsgasen eigens für diesen Zweck installierter Brenner oder durch elektrische Heizung erfolgen. In einer bevorzugten Ausführungsform des neuen Verfahrens, wird das frische, flüssige 1,2-Dichlorethan, bevor es in den zweiten Behälter eingespeist wird, in der Konvektionszone des Spaltofens mit dem Rauchgas, das die den Spaltofen heizenden Brenner erzeugen, erwärmt. In einer weiteren bevorzugten Ausführungsform des neuen Verfahrens, wird das frische, flüssige 1,2-Dichlorethan mit einem Temperiermedium erwärmt, das seinerseits in der Konvektionszone des Spaltofens mit dem Rauchgas, das die den Spaltofen heizenden Brenner erzeugen, erwärmt wird. Als Temperiermedium sind hierfür, wie oben bereits erwähnt, erhitzte hochsiedende Flüssigkeiten, wie Mineralöl, Siliconöl oder geschmolzenes Diphenyl sowie insbesondere Wasserdampf geeignet.

Das 1,2-Dichlorethan wird in einem ersten Behälter mit Vinylchlorid enthaltendem Gas bis zum Sieden indirekt erwärmt. Zweckmäßig wird hierzu ein Wärmetauscher benützt, indem das heiße Vinylchlorid enthaltende Gas aus dem Spaltofen durch mindestens ein Rohr strömt, das im wesentlichen gerade sein kann oder schraubenförmig, spiralförmig sowie mäanderförmig gebogen sein kann. Dieses Rohr ist von dem flüssigen zu erwärmenden 1,2-Dichlorethan umgeben. Es ist vorteilhaft, wenn während der indirekten Erwärmung des 1,2-Dichlorethans im ersten Behälter das heiße, Vinylchlorid enthaltende Gas aus dem Spaltofen mit einer mittleren Abkühloeschwindigkeit von mindestens pro Sekunde 1/15 der Temperatur in Grad Celsius, mit der dieses Gas in die Zone der indirekten Erwärmung des 1,2-Dichlorethans eintritt, bis zum Erreichen einer Temperatur,die mindestens 5 °C über der Verdampfungstemperatur des 1,2-Dichlorethans im zweiten Behälter liegt, abgekühlt wird. Wenn beispielsweise das heiße Vinylchlorid enthaltende Gas mit einer Temperatur von 525 °C in den ersten Behälter eintritt, so sollte die mittlere Abkühlgeschwindigkeit mindestens 525 : 15 = 35 °C pro Sekunde betragen. Verdampftdas 1,2-Dichlorethan im zweiten Behälter beispielsweise bei 260 °C, so soll die Temperatur des Vinylchlorid enthaltenden Gases beim Verlassen des ersten Behälters mindestens 265 °C betragen. Die Abkühlgeschwindigkeit des Vinylchlorid enthaltenden Gases im ersten Behälter kann sehr hoch sein. Bei mehr als pro Sekunde 1/5 der Temperatur in Grad Celsius, mit der dieses Gas in die Zone der indirekten Erwärmung des 1,2-Dichlorethans eintritt, wird es im allgemeinen technisch immer schwieriger, den nötigen Wärmeübergang für so hohe Abkühlgeschwindigkeiten zu erzielen. Die Temperatur, mit der das Vinylchlorid enthaltende Gas die Zone der indirekten Erwärmung des 1,2-Dichlorethans verläßt, ist nach oben selbstverständlich begrenzt durch die Eintrittstemperatur des Vinylchlorid enthaltenden Gases in diese Zone, im allgemeinen wird sie jedoch aus wirtschaftlichen Erwägungen nicht mehr als 50 °C über der Verdampfungstemperatur des 1,2-Dichlorethans im zweiten Behälter liegen.

Im ersten Behälter wird das 1,2-Dichlorethan bis zum Sieden erwärmt. Das so erwärmte 1,2-Dichlorethan wird in einen zweiten Behälter übergeführt, in dem es ohne weitere Erwärmung unter geringerem Druck als im ersten Behälter teilweise verdampft. Diese Strömung des flüssigen, erwärmten 1,2-Dichlorethans vom ersten in den zweiten Behälter sowie auch die des nicht-verdampften 1,2-Dichlorethans vom zweiten zurück in den ersten Behälter erfolgt vorteilhaft ohne Anwendung mechanischer Fördermittel durch einen sogenannten "Naturumlauf". Dieser kommt dadurch zustande, daß das am Siedepunkt befindliche 1,2-Dichlorethan in mindestens einem Rohr von dem ersten in den darüberliegenden zweiten Behälter aufsteigt, getrieben durch den Effekt, daß das anfänglich relativ wenige Dampfblasen enthaltende flüssige 1,2-Dichlorethan spezifisch leichter ist, als die keine Dampfblasen enthaltende Flüssigkeit, wodurch sie sich im ersten Behälter oben sammelt und durch ein nach oben führendes Rohr austritt. Während des Weges nach oben nimmt der Druck ab, wodurch immer mehr 1,2-Dichlorethan verdampft. Dies führt zu einer ständigen Volumen-Zunahme des Flüssigkeits-Dampfgemisches und Abnahme des spezifischen Gewichtes dieser Mischung. Schließlich gelangt das Flüssigkeits-Dampfgemisch in den zweiten, oberen Behälter, in dem es sich in die beiden Phasen trennt. Die Dampfphase wird am oberen Teil des zweiten Behälters abgeführt und in die Spaltzone des Pyrolyseofens eingeleitet. Das nichtverdampfte, flüssige 1,2-Dichlorethan sammelt sich im Unterteil des zweiten, oberen Behälters, von dem aus mindestens eine Leitung in den unteren Teil desersten Behälters zurückführt. In dieser Leitung fließt die keine Dampfblasen enthaltende Flüssigkeit, die durch Zufuhr kühleren, frischen 1,2-Dichlorethans in den zweiten, oberen Behälter kälter und damit spezifisch schwerer ist, in den ersten, unteren Behälter zurück. Hier wird si e durch das heiße, vinylchlorhaltige Gas zum Sieden erwärmt und der Kreislauf beginnt von neuem. Die Umlaufmenge ($m_u$) des 1,2-Dichlorethans kann aus der zugeführten Menge frischen 1,2-Dichlorethans ($m_o$), dessen Temperatur ($t_o$) sowie der Temperatur der Flüssigkeit, die in der Leitung von dem zweiten, oberen Behälter in den ersten,unteren Behälter strömt ($t_l$) und der Tem-

peratur, der vom ersten in den zweiten Behälter aufsteigenden Flüssigkeit ($t_2$) nach folgender Formel berechnet werden:

$$m_u = \frac{m_o \cdot (t_1 - t_o)}{(t_2 - t_1)}$$

Die Menge des in den zweiten Behälter eingespeisten, frischen 1,2-Dichlorethans, bezogen auf 100 kg pro Stunde, des zwischen dem ersten und dem zweiten Behälter umlaufenden 1,2-Dichlorethans, kann in weiten Grenzen schwanken, vorteilhaft werden 2 bis 20 kg und insbesondere 3 bis 10 kg pro Stunde, frisches 1,2-Dichlorethan, bezogen auf 100 kg pro Stunde umlaufendes 1,2-Dichlorethan in den zweiten Behälter eingeführt.

In einer bevorzugten Ausführungsform des erfindungsgemäßen Verfahrens, wird ein Teil des flüssigen 1,2-Dichlorethans aus dem ersten Behälter, in dem es bis zum Sieden erwärmt wird, abgezogen, falls erforderlich, von festen Bestandteilen getrennt und in eine Destillationskolonne eingespeist. Vorteilhaft wird hierzu eine Destillationskolonne verwendet, in der über Kopf 1,2-Dichlorethan abdestilliert wird und die ohnehin vorhanden ist, um das thermisch nicht gespaltene 1,2-Dichlorethan vor der Wiederverwendung zu reinigen. In diesem Fall brauchen, wenn überhaupt, nur gröbere, feste Bestandteile abgetrennt zu werden, was beispielsweise mit einem einfachen Sieb bewerkstelligt werden kann. Es hat sich als besonders günstig erwiesen je 100 kg pro Stunde des in den zweiten Behälter frisch eingespeisten, flüssigen 1,2-Dichlorethans 0,5 bis 7 kg pro Stunde flüssiges 1,2-Dichlorethan aus dem ersten Behälter abzuführen.

Die Temperatur, bei der das im zweiten Behälter verdampfte 1,2-Dichlorethan diesen Behälter verläßt, kann in erheblichen Grenzen schwanken. Vorteilhaft beträgt diese Temperatur 170 bis 280 °C, insbesondere 220 bis 280 °C. Diese Temperatur wird zweckmäßig eingestellt durch Regelung des Drucks am Kopf einer Kolonne zur Abtrennung von Chlorwasserstoff aus dem Vinylchlorid enthaltenden Gas, das nach dem indirekten Wärmetausch den ersten Behälter verläßt, wobei die Kopftemperatur dieser Kolonne -20 bis -50 °C beträgt.

Die mittlere Verweilzeit des 1,2-Dichlorethans im ersten und zweiten Behälter zusammen sollte insbesondere bei höheren Verdampfungstemperaturen des 1,2-Dichlorethans nicht zu lang sein, da dies die Bildung von Nebenprodukten begünstigt.Vorteilhaft wird bei mittleren Verweilzeiten von 15 bis 90 Minuten gearbeitet, es sind vor allem bei niedrigeren Verdampfungstemperaturen aber auch noch längere Verweilzeiten möglich, jedoch auch aus wirtschaftlichen Gründen oft nicht wünschenswert. Um möglichst hohe Spaltumsätze bei gleichbleibender Zusammensetzung der Spaltprodukte zu erzielen, ist es wünschenswert, das im zweiten Behälter verdampfte 1,2-Dichlorethan in möglichst konstanter Menge je Stunde in den Spaltofen einzuspeisen. Dies ist mit dem neuen Verfahren wegen seiner günstigen Regelunosmöglichkeiten besonders gut erreichbar.

Die Temperatur der Rohre im Spaltofen, in denen das 1,2-Dichlorethan in Vinylchorid und Chlorwasserstoff gespalten wird, wird durch Regelung der Brennstoffzufuhr zu diesem Ofen zweckmäßig so eingestellt, daß 60 bis 70 Gew.-% des in diesen Ofen eingespeisten erfindungsgemäß verdampften 1,2-Dichlorethans thermisch gespalten werden. Günstig ist hierbei, den üblicherweise verwendeten Spaltofen mit mehreren übereinanderliegenden Brennerreihen so zu beheizen, daß auf je ein Kilogramm Brennstoff, mit dem die unteren Brennerreihen beaufschlagt werden, 1 bis 2,3 kg Brennstoff den oberen Brennerreihen zugeführt werden. Es ist jedoch auch eine andere Befeuerung des Spaltofens möglich.

Das im ersten Behälter verdampfte 1,2-Dichlorethan kann sowohl in die Strahlungszone wie auch in die Konvektionszone eines üblichen Spaltofens eingeleitet werden, wobei mit "Strahlungszone" der Teil des Ofens bezeichnet ist, in dem das in mindestens einem Rohr geführte 1,2-Dichlorethan der direkten Strahlungswärme der Brennerflammen, die den Ofen beheizen, ausgesetzt ist, während "Konvektionszone" den Teil des Ofens bezeichnet, in dem das in mindestens einem Rohr geführte 1,2-Dichlorethan im wesentlichen nur noch durch die heißen Rauchgase, die die Brenner erzeugen, erwärmt wird. Vorteilhaft wird das verdampfte 1,2-Dichlorethan am Beginn der Strahlungszone in den Spaltofen eingespeist.

Es kann jedoch in bestimmten Fällen, in Abhängigkeit von der Ofenkonstruktion und der Beheizung, auch vorteilhaft sein, die Einspeisung in den Teil der Konvektionszone zu verlegen, der an die Strahlungszone angrenzt und den restlichen Bereich der Konvektionszone beispielsweise zur Vorwärmung des flüssigen 1,2-Dichlorethans vor der Verdampfung zu verwenden.

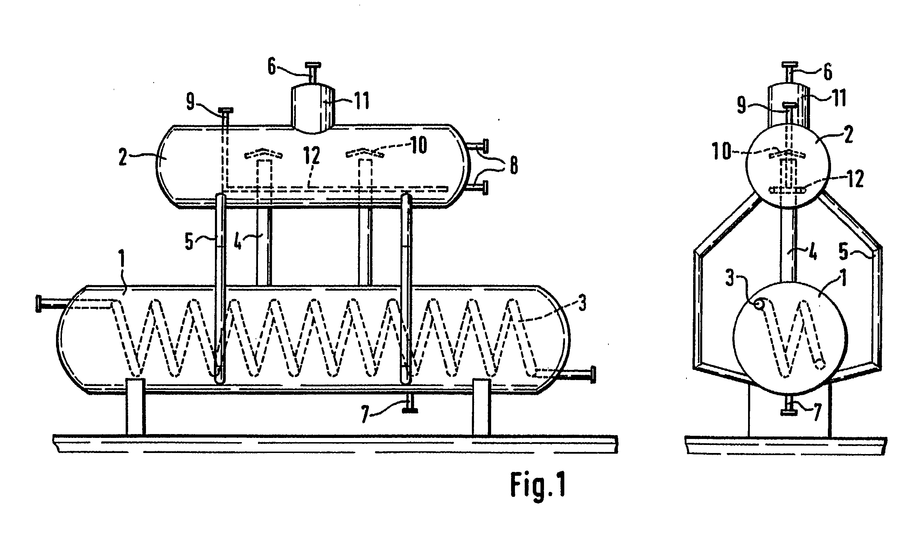

Gegenstand der Erfindung ist auch eine Vorrichtung zur Durchführung des weiter oben beschriebenen Verfahrens, die beispielhaft in Figur 1 dargestellt ist. Die verschiedenen Apparateteile in dieser Figur sind mit Zahlen bezeichnet, auf die sich die nachstehend in Klammern aufgeführten Zahlen beziehen. Die Vorrichtung besteht aus zwei geschlossenen, zylindrischen Behältern (1; 2), deren Verhältnis von Länge zu Durchmesser 2 bis 8 beträgt, die miteinander durch Rohre verbunden sind und von denen ein Behälter eine Rohrschlange (3) enthält, und die dadurch gekennzeichnet ist, daß beide Behälter (1; 2) mit waagerechter oder gegen die Waagerechte leicht geneigter Zylinderachse weitgehend parallel übereinander in einem Abstand angeordnet sind, der untere Behälter (1) die Rohrschlange (3) enthält, vom obersten Teil des unteren Behälters (1) mindestens ein Steigrohr (4) in den oberen Behälter (2) führt und dort in der oberen Hälfte offen endet, vom unteren Teil des oberen Behälters (2) zum unteren Teil des unteren Behälters (1) mindestens ein Verbindungsrohr (5) führt, der untere Behälter (1) im unteren Teil und der obere Behälter (2) im oberen Teil je eine Öffnung (6; 7) enthält und der obere Behälter (2) eine Flüssigkeits-Standhöhenmessung (8) sowie mindestens eine weitere Öffnung (9), von der ein Rohr in den unteren Teil dieses Behälters führt, aufweist. Die beiden zylindrischen Behälter sind mit waagerechter Zylinderachse angeordnet, um eine größere Flüssigkeits-Oberfläche im Vergleich zu einem Behälter mit senkrecht stehender Zylinderachse zu erhalten. Aus dem gleichen Grund sollte das Verhältnis von Länge zu Durchmesser bei beiden Gefäßen nicht wesentlich unter 2 liegen. Das erfindungsgemäße Verfahren kann auch in sehr schlanken Behältern mit einem hohen Verhältnis von Länge zu Durchmesser durchgeführt werden, allerdings sprechen wirtschaftliche und konstruktive Bedenken dagegen, ein zu hohes Verhältnis zu wählen. Im allgemeinen wird man 8 nicht überschreiten. Der obere Behälter (2) braucht nicht unbedingt genau senkrecht über dem unteren Behälter (1) angeordnet zu sein, auch ist es nicht erforderlich, den oberen Behälter (2) kleiner als den unteren auszuführen. Der obere Behälter enthält zweckmäßig einen Dom (11), auf dem die Öffnung (6) angebracht ist. Die Steigrohre (4) sowie die Verbindungsrohre (5) sollten etwa gleichmäßig verteilt über die Länge beider Behälter angeordnet sein, wobei für die Steigrohre (4) günstig ein größerer Querschnitt gewählt wird als für die Verbindungsrohre (5). Anzahl und Querschnitt beider Rohrarten richten sich nach der Länge der Behälter (1; 2) sowie nach der Menge des in beiden Behältern umlaufenden flüssigen 1,2-Dichlorethans, deren Berechnung weiter oben beschrieben ist. Es ist ferner nicht unbedingt erforderlich, die Verbindungsrohre (5) paarweise gegenüberliegend anzuordnen.

Vorteilhaft ist das obere Ende des Steigrohres beziehungsweise der Steigrohre (4) mit einer Haube (10) so bedeckt, daß zwischen dieser und dem Rohrende eine ringförmige Öffnung freibleibt. Hierdurch wird die Trennung der flüssigen von der gasförmigen Phase im oberen Behälter (2) verbessert.

Der obere Behälter (2) kann eine oder mehrere Öffnungen (9) aufweisen, von denen Rohre in den unteren Teil dieses Behälters führen. In einer bevorzugten Ausführungsform der erfindungsgemäßen Vorrichtung wird nur eine solche Öffnung verwendet, von der ein Rohr in den unteren Teil des Behälters führt, wobei am Ende dieses Rohres eine waagerecht liegende, am Ende geschlossene Rohrschleife (12) angebracht ist, die auf ihrer Länge gleichmäßig verteilt Öffnungen enthält.

Wie oben bereits erwähnt, ermöglicht es das neue Verfahren, die in den vinylchloridhaltigen Gasen, die den Spaltofen verlassen, enthaltene Wärme wieder zu verwenden und somit Energie einzusparen. Das Verfahren kann in einer Vorrichtung durchgeführt werden, die keine störanfälligen Teile (Pumpe und Filter für heißes 1,2-Dichlorethan) enthält. Durch günstige Regelungsmöglichkeiten kann das neue Verfahren an Produktionsschwankungen flexibel angepaßt werden, bei vergleichbarer Spaltofen-Laufzeit kann ein deutlich höherer Spaltumsatz gefahren werden, als nach entsprechenden Verfahren nach dem Stande der Technik.

Nachfolgende Beispiele sollen die Erfindung näher erläutern, sie ist jedoch nicht auf diese Beispiele beschränkt.

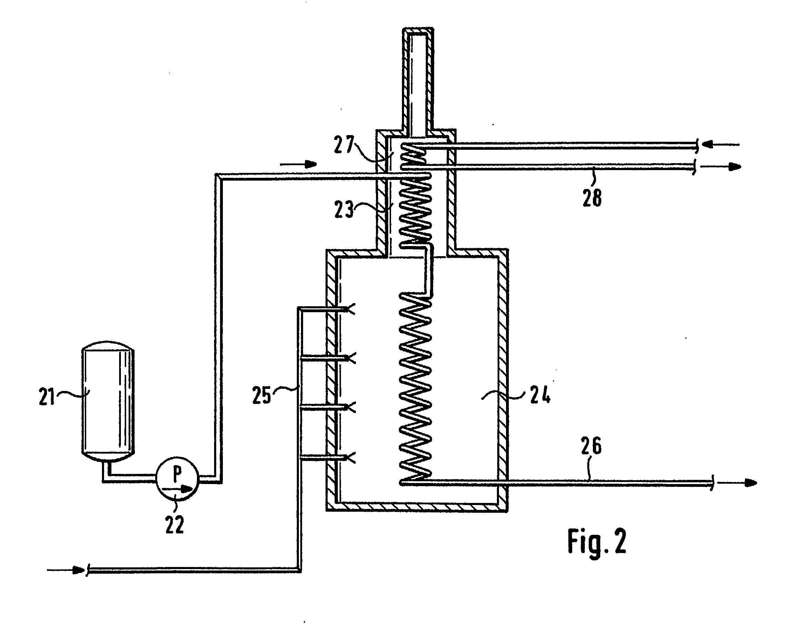

Zum Vergleich wird zunächst nach einem Verfahren gearbeitet, das auch großtechnisch zur Herstellung von Vinylchlorid durch thermische Spaltung von 1,2-Dichlorethan verwendet wird, siehe hierzu, das in Figur 2 gezeigte Fließschema.

Aus einer Pumpenvorlage (21) werden stündlich 902 kg 1,2-Dichlorethan mit einer Temperatur von 130 °C abgezogen und mit der Pumpe (22) unter einem Druck von 3,0 MPa und einer Temperatur von 125 °C in die Konvektionszone (23) des Spaltofens (23 plus 24) gefördert.

In dieser Zone wird 1,2-Dichlorethan bis zum Siedepunkt erhitzt, verdampft und mit einer Temperatur von 270 °C in die Strahlungszone (24) des Spaltofens (23 plus 24) weitergeführt. In der Strahlungszone (24) erfolgt die Überhitzung und partielle Spaltung des gasförmigen Dichlorethans zu Vinylchlorid und Chlorwasserstoff bis zu einer Temperatur von 533 °C. Die Strahlungszone (24) enthält vier übereinanderliegende Brenner-Reihen (25) von denen jedoch nur die drei unteren Reihen mit Brennstoff beaufschlagt werden. Um die Rußbildung bei der 1,2-Dichlorethan-Verdampfung zu verringern, bleibt die vierte, oberste Brenner-Reihe außer Betrieb.

Die den Spaltofen über die Leitung (26) verlassenden, heißen Spaltgase, werden unter einem Druck von 1,6 MPa in der nachfolgenden Kühlung auf eine Temperatur von kleiner als 100 °C abgeschreckt. Nach weiteren Kühlstufen erfolgt die Abtrennung von Chlorwasserstoff aus dem durch thermische Spaltung entstandenen Stoffgemisch in einer Kolonne, die unter einem Druck von 1,3 MPa und einer Kopftemperatur von -24 °C arbeitet.

Durch den Wärmetausch in der Konvektionszone (23) zwischen Rauchgas, das um die Rohre geführt wird und 1,2-Dichlorethan in den Rohren, kühlt sich das Rauchgas auf etwas 350 °C ab. In einem Economizer (27) erfolgt durch Erzeugung von Heißwasser eine weitere Abkühlung des Rauchgases auf etwa 150 °C. Dabei können pro Stunde 480 dm³ Kesselspeisewasser unter einem Druck von 2,5 MPa von 80 °C auf 150 °C erwärmt werden. Das erwärmte Kesselspeisewasser wird über die Leitung (28) der Dampferzeugung zugeführt. Dieser Wasserdampf wird an anderer Stelle im Vinylchlorid-Herstellungsverfahren verwendet.

Es werden 312,5 kg/h Vinylchlorid erzeugt, der Spaltumsatz beträgt 55 %, die Spaltofenlaufzeit maximal 6 Monate. Es werden je 1 kg erzeugtes Vinylchlorid 0,115 Nm³ Brennstoff (Methan) verwendet. Die aus den Rauchgasen durch Erzeugung von Heißwasser zurückgewonnene Energie beträgt 448,2 kJ/kg Vinylchlorid, entsprechend einer Brennstoffmenge von 0,0126 Nm³/kg Vinylchlorid. Somit reduziert sich der effektive Brennstoffverbrauch auf 0,1024 Nm³/kg Vinylchlorid.

Das zum Einsatz kommende 1,2-Dichlorethan hat eine Reinheit von 99,731 Gew.-%, der Rest sind Nebenprodukte. Für diesen Vergleichsversuch sowie für die nachfolgenden Beispiele wird ein 1,2-Dichlorethan gleicher Reinheit und nach Art und Menge gleicher Zusammensetzung des geringen Anteils der Nebenprodukte verwendet.

Beispiel 1:

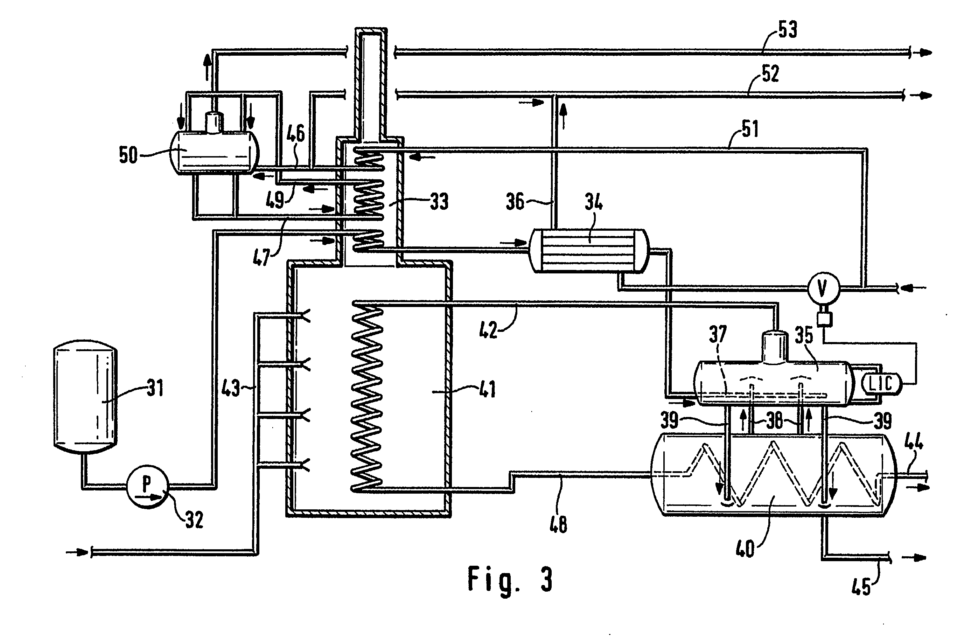

Es wird nach dem in Figur 3 dargestellten Fließschema gearbeitet. Aus einer Pumpenvorlage (31) werden stündlich 834 kg 1,2-Dichlorethan mit einer Temperatur von 130 °C abgezogen und mit der Pumpe (32) unter einem Druck von 4,0 MPa undeiner Temperatur von 125 °C in den unteren Bereich der Konvektionszone (33) des Spaltofens (33 plus 41) gefördert. Durch die abziehenden Rauchgase aus der Strahlungszone (41) des Spaltofens wird das flüssige 1,2-Dichlorethan auf 220 °C erwärmt, wobei sich die Rauchgase von 930 °C auf 710 °C abkühlen. Im Wärmetauscher (34) erfolgt der Energieausgleich zwischen den, an das flüssige 1,2-Dichlorethan Energie abgebenden Bereich (33) und der für die 1,2-Dichlorethan-Verdampfung benötigten Energieaufnahme im ersten Behälter (40). Zu diesem Zweck wird die Standhöhe des flüssigen 1,2-Dichlorethans im zweiten Behälter (35) mit einer üblichen Einrichtung (LIC) gemessen und mit diesem Meßwert als Regelgröße die erforderliche Menge des unter einem Druck von 2,5 MPa stehenden Kesselspeisewassers als Kühlmittel dem Wärmetauscher (34) zugeführt. Für die Abkühlung sind 210 dm³ des Kesselspeisewassers erforderlich, das sich dabei von 80 °C auf 150 °C erwärmt und den Wärmetauscher (34) über die Leitung (36) verläßt. Die rückgewonnene Energiemenge beträgt 185,7 kJ/kg Vinylchlorid.

Das auf etwa 185 °C abgekühlte 1,2-Dichlorethan wird über eine Rohrschleife mit gleichmäßig verteilten Öffnungen (37) dem zweiten Behälter (35) zugeführt und mischt sich dort mit dem heißeren 1,2-Dichlorethan, das vom ersten Behälter (40) durch die Rohre (38) in den zweiten Behälter aufgestiegen ist, wobei ein Teil dieses 1,2-Dichlorethans verdampft . Im ersten Behälter (40) wird flüssiges 1,2-Dichlorethan durch Wärmetausch mit dem die Spaltungszone (41) des Spaltofens (33 plus 41) durch die Leitung (48) verlassenden Vinylchlorid enthaltenden, heißen Gas bis zum Sieden erwärmt. Der Wärmetausch wird begünstigt durch den weiter oben näher erläuterten "Naturumlauf" des 1,2-Dichlorethans zwischen dem ersten Behälter (40) und dem zweiten Behälter (35) über die aufsteigenden Rohre (38) und nach unten führenden Rohre (39). Das Flüssigkeits-Gasgemisch, in den aufsteigenden Rohren (38) hat eine Temperatur von 270 °C , die Flüssigkeit in den nach unten führenden Rohren (39) hat eine Temperatur von 265 °C. Nach der weiter oben angegebenen Gleichung beträgt die Menge des zwischen dem ersten und zweiten Behälter umlaufenden 1,2-Dichlorethans 13 344 kg/h. Je 100 kg/h des zwischen dem ersten Behälter (40) und zweiten Behälter (35) umlaufenden,flüssigen 1,2-Dichlorethans, werden 6,25 kg/h frisches, flüssiges 1,2-Dichlorethan in den zweiten Behälter (35) eingespeist. Das im wesentlichen in den aufsteigenden Rohren (38) und dem zweiten Behälter (35) verdampfte 1,2-Dichlorethan wird frei von flüssigen oder festen Bestandteilen über die Leitung (42) in die Strahlungszone (41) des Spaltofens (33 plus 41) eingeführt, in der das gasförmige 1,2-Dichlorethan mit vier übereinanderliegenden Brenner-Reihen (43) auf 533 °C erhitzt wird. Die unteren und oberen Brenner-Reihen werden mit der gleichen Menge Brennstoff beaufschlagt.

Während der Überhitzung des Gases auf 533 °C wird ein Teil des 1,2-Dichlorethans in Vinylchlorid und Chlorwasserstoff gespalten. Die heißen Spaltgase werden, wie oben bereits erwähnt, dem ersten Behälter (40) über die Leitung (48) zugeführt und verlassen diesen Behälter mit einer Temperatur von 275 °C. Die durchschnittliche Abkühlgeschwindigkeit der Spaltgase im ersten Behälter (40) beträgt 46 °C/s, das sind 1/11,6 der Eingangs-Temperatur (533 °C) pro Sekunde. Über die Leitung (44) werden diese Spaltgase einer weiteren Kühlung nach dem Stande der Technik (nicht in Figur 3 dargestellt) zugeführt, wobei sie teilweise kondensieren. Aus dem durch thermische Spaltung entstandenen Stoffgemisch wird nach bekanntem Verfahren in einer Kolonne (ebenfalls nicht in Figur 3 dargestellt) Chlorwasserstoff bei einer Kopftemperatur von -24 °C abgetrennt. Der Druck am Kopf dieser Kolonne wird so eingestellt, daß das gasförmige verdampfte 1,2-Dichlorethan mit einer Temperatur von 270 °C den zweiten Behälter (35) verläßt. In diesem Behälter verdampfen 804 kg/h 1,2-Dichlorethan bei einem Druck von 3,7 MPa. Je Quadratmeter Oberfläche der im zweiten Behälter (35) als ruhend angenommenen Flüssigkeits-Oberfläche werden 2 880kg/h 1,2-Dichlorethan verdampft. Aus dem Unterteil des ersten Behälters (40) werden 30 kg/h flüssiges 1,2-Dichlorethan abgezogen und über die Leitung (45) einer Kolonne, in der 1,2-Dichlorethan über Kopf abdestilliert wird (nicht in Figur 3 dargestellt) zugeführt. Das sind 3,6 kg/h aus dem ersten Behälter (40) abgezogenes 1,2-Dichlorethan je 100 kg/h des in den zweiten Behälter (35) frisch eingespeisten 1,2-Dichlorethans. Die mittlere Verweilzeit des 1,2-Dichlorethans, im ersten und zweiten Behälter zusammen, beträgt 47 Minuten.

Die Brenner im Spaltofen werden über die Leitung (43) mit 0,103 Nm³ Brennstoff (Methan) je kg erzeugtes Vinylchlorid beaufschlagt. Die heißen Rauchgase, welche die 1,2-Dichlorethan-Vorwärmzone mit 710 °C verlassen, werden durch Dampf- und Heißwassererzeugung vor Eintritt in die Atmosphäre auf 150 °C abgekühlt. Im oberen Abschnitt der Konvektionszone (33) des Spaltofens wird über die Leitung (51) zugeführtes, kühleres Wasser erwärmt und über die Leitung (46) teilweise dem Kessel (50) zugeführt, teilweise über die Leitung (52) an anderen Stellen des Prozesses verwendet. Das heiße Wasser aus dem Kessel (50) wird über die Leitung (47) in den mittleren Teil der Konvektionszone (33) des Spaltofens eingespeist und nach Wärmeaufnahme aus den aufsteigenden Rauchgasen über die Leitung (49) dem Kessel (50) wieder zugeführt. Der dort erzeugte Wasserdampf wird über die Leitung (53) abgeführt und an anderen Stellen des Vinylchlorid-Herstellungsprozesses verwendet. Es werden 114 kg/h Hochdruckdampf (2,1 MPa, 215 °C) erzeugt und über die Leitung (53) abgegeben. Die hierbei zurückgewonnene Energie beträgt 843,9 kJ/kg Vinylchlorid. Über die Leitung (52) werden 201 dm³/h Heißwasser von 150 °C abgegeben, die hierbei rückgewonnene Energiemenge beträgt 178,8 kJ/kg Vinylchlorid.

Der Umsatz bei der Spaltung des 1,2-Dichlorethans in der Strahlungszone (41) des Spaltofens (33 plus 41) beträgt 65 %. Es werden 330 kg/h Vinylchlorid produziert.

Nach 9 Monaten Laufzeit ist der Wärmeübergang zwischen den heißen Vinylchlorid enthaltenden Gasen aus dem Spaltofen und dem flüssigen 1,2-Dichlorethan im ersten Behälter (40) nahezu unverändert. Die Temperaturdifferenz zwischen den heißen Gasen aus dem Spaltofen, die über die Leitung (44) aus dem ersten Behälter (40) abgeführt werden und dem gasförmigen 1,2-Dichlorethan, das den zweiten Behälter (35) verläßt und über die Leitung (42) dem Spaltofen zugeführt wird, beträgt 10 °C. Auf den Wärmetauschflächen wird weder auf der Seite der heißen Spaltgase noch auf der Seite des flüssigen 1,2-Dichlorethans ein nennenswerter Belag festgestellt. Die aus den Rauchgasen des Spaltofens durch Erzeugung von Heißwasser und Hochdruckdampf zurückgewonnene Energie beträgt 185,7 + 178,8 + 843,9 = 1 208,4 kJ/kg Vinylchlorid, dies entspricht einer Brennstoff (Methan)-Menge von 0,034 Nm³/kg Vinylchlorid. Damit reduziert sich der effektive Heizgasverbrauch auf 0,069 Nm³/kg Vinylchlorid, daß sind nur 67,4 % von der Menge (100 %), die sich aus dem Vergleichsversuch ergibt. Die Energieeinsparung beträgt demnach 32,6 %,neben einer Vergrößerung des Spaltumsatzes von 55 auf 65 % und einer Verlängerung der Laufzeit des Spaltofens von 6 auf 9 Monate.

Beispiel 2:

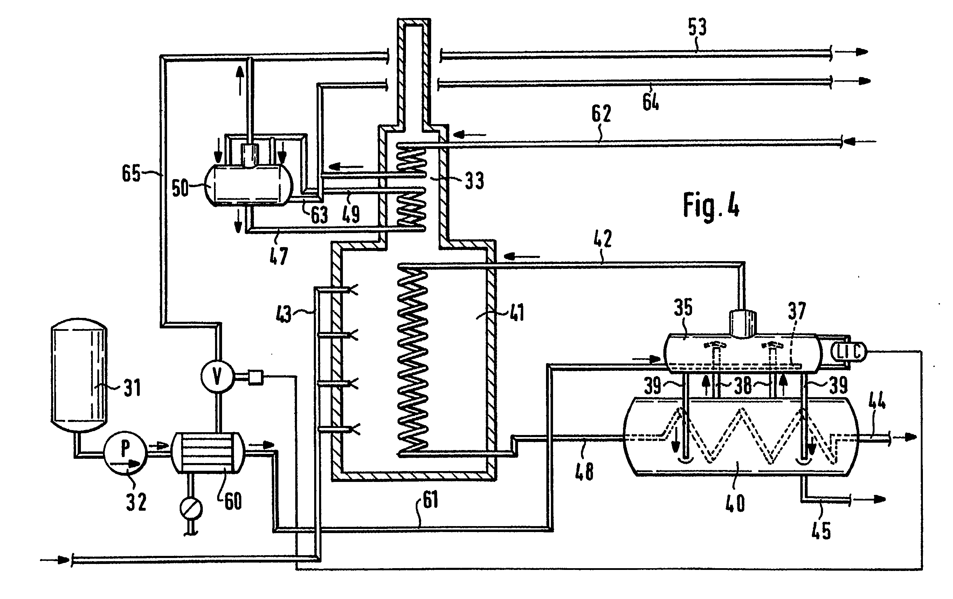

Es wird nach dem in Figur 4 dargestellten Fließschema gearbeitet. Die in diesem Schema mit gleichen Zahlen wie in Figur 3 bezeichneten Apparateteile sind bereits im Beispiel 1 beschrieben. Die Verfahrensweise im Beispiel 2 unterscheidet sich von der im Beispiel 1 lediglich in folgenden Punkten:

Aus einer Pumpenvorlage (31) werden stündlich 834 kg 1,2-Dichlorethan mit einer Temperatur von 130 °C abgezogen und mit der Pumpe (32) unter einem Druck von 2,9 MPa und einer Temperatur von 125 °C über den Wärmetauscher (60) über die Leitung (61) ohne Erwärmung in der Konvektionszone (33) des Spaltofens (33 plus 41) direkt in den zweiten Behälter (35) gefördert. Der Wärmetauscher (60) wird mit 25 kg/h Hochdruckdampf (2,1 MPa Druck; 215 °C) aus dem Kessel (50) über die Leitung (65) geheizt. Über die Messung der Standhöhe des flüssigen 1,2-Dichlorethans (LIC) im zweiten Behälter (35) als Regelgröße, wird die Hochdruckdampfzufuhr zum Wärmetauscher (60) geregelt. Das 1,2-Dichlorethan verläßt den Wärmetauscher (60) mit einer Temperatur von 161 °C. Die heißen, vinylchloridhaltigen Spaltgase verlassen die Strahlungszone (41) des Spaltofens (33 plus 41) über die Leitung (48) mit einer Temperatur von 533 °C, durchlaufen den ersten Behälter (40) und verlassen diesen mit einer Temperatur von 245 °C. Die Abkühlgeschwindigkeit der heißen Spaltgase im ersten Behälter (40) beträgt 51,7 °C/s, daß sind 1/10,3 der Eintrittstemperatur (533 °C) in diesen Behälter pro Sekunde. Nach Verlassen des ersten Behälters werden die Spaltgase nach üblichem Verfahren weiter abgekühlt und in einer Kolonne mit einer Kopftemperatur von -31 °C Chlorwasserstoff abdestilliert. Der Druck am Kopf dieser Kolonne wird so eingestellt, daß das 1,2-Dichlorethan den zweiten Behälter (35) bei einem Druck von 2,6 MPa mit einer Temperatur von 240 °C verläßt. Es verdampfen in diesem Behälter und in den Steigrohren (38) 804 kg/h 1,2-Dichlorethan und werden über die Leitung (42) der Strahlungszone (41) des Spaltofens zugeführt.

Die Temperatur in den Steigrohren (38) beträgt 240 °C, die in den nach unten führenden Rohren (39) 235 °C. Nach der weiter oben beschriebenen Gleichung errechnet sich die zwischen dem ersten (40) und zweiten (35) Behälter umlaufende Menge flüssiges 1,2-Dichlorethan zu 13 177 kg/h. Je 100 kg/h zwischen dem ersten und dem zweiten Behälter umlaufenden 1,2-Dichlorethans werden 6,33 kg/h frisches 1,2-Dichlorethan in den zweiten Behälter (35) eingespeist. Am Boden des ersten Behälters (40) werden über die Leitung (45) 30 kg/h flüssiges 1,2-Dichlorethan abgezogen und einer Kolonne zugeführt, in der über Kopf 1,2-Dichlorethan abdestilliert wird, das sind je 100 kg/h in den zweiten Behälter (35) eingespeisten 1,2-Dichlorethans 3,6 kg/h. Die mittlere Verweilzeit des 1,2-Dichlorethans, im ersten und zweiten Behälter zusammen, beträgt 47 Minuten, je m² der als ruhend angenommenen Flüssigkeitsoberfläche im zweiten Behälter (35) werden 2 880 kg/h 1,2-Dichlorethan verdampft.

Die vier übereinanderliegenden Brenner-Reihen des Spaltofens (33 plus 41) werden über die Leitung (43) mit insgesamt 0,1074 Nm³ Brennstoff (Methan) je Kilogramm erzeugtes Vinylchlorid beaufschlagt. Im oberen Teil der Konvektionszone (33) des Spaltofens (33 plus 41) werden in einem Economizer 330 dm³/h Kesselspeisewasser (Druck 2,5 MPa), das über die Leitung (62) mit 80 °C zugeführt wird, auf 150 °C erwärmt und teilweise über die Leitung (63) in den Kessel (50) eingespeist, teilweise über die Leitung (64) an anderer Stelle des Vinylchlorid-Herstellungsprozesses wiederverwendet. Wie im Beispiel 1 wird die Flüssigkeit aus dem Kessel (50) in dem unteren Teil der Konvektionszone (33) erwärmt und dem Kessel (50) über die Leitung (49) zugeführt. Ein Teil des im Kessel (50) erzeugten Dampfes wird, wie oben bereits erwähnt, zur Heizung des Wärmetauschers (60) verwendet. Der größere Teil dieses Dampfes, nämlich 167 kg/h, wird an anderen Stellen des Prozesses zur Erzeugung von Vinylchlorid genutzt.

Hierdurch werden 1 236,2 kJ/kg Vinylchlorid an Energie wiedergewonnen. Durch die Leitung (64) werden 136 dm³/h Kesselspeisewasser mit einer Temperatur von 150 °C der weiteren Verwendung zugeführt, wodurch 121 kJ/kg Vinylchlorid Energie zurückgewonnen werden. Die gesamte wiedergewonnene Energiemenge beträgt 1 236,2 + 121 = 1 357,2 kJ/kg Vinylchlorid, dies entspricht einer Brennstoff-(Methan)-Menge von 0,038 Nm³/kg Vinylchlorid. Der effektive Heizgasverbrauch reduziert sich damit auf 0,0694 Nm³/kg, dies sind nur 67,8 % des Verbrauches (100 %), der im Vergleichsversuch erforderlich war. Die Energieeinsparung beträgt demnach 32,2 %. Wie im Beispiel 1 werden nach 9 Monaten Laufzeit noch keine nennenswerten Beläge an den Wärmeaustauschflächen im ersten Behälter (40) festgestellt, die Spaltofen-Laufzeit beträgt ebenfalls mindestens 9 Monate. Es werden 330 kg/h Vinylchlorid produziert, der Umsatz bei der Spaltung des 1,2-Dichlorethans beträgt 65 %.

Beispiel 3:

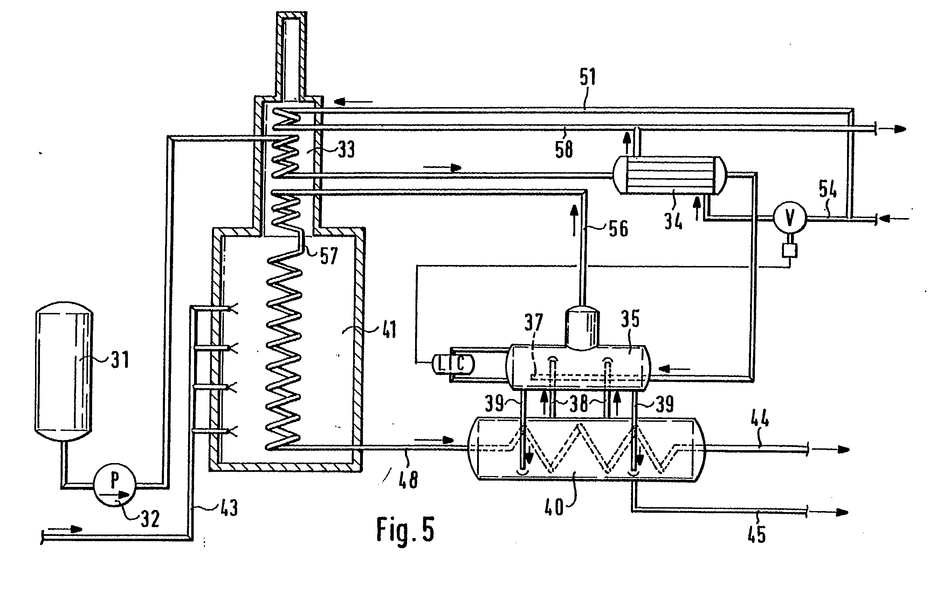

Es wird nach dem in Figur 5 dargestellten Fließschema gearbeitet. Aus einer Pumpenvorlage (31) werden stündlich 785 kg 1,2-Dichlorethan mit einer Temperatur von 130 °C abgezogen und mit der Pumpe (32) unter einem Druck von 3,6 MPa und einer Temperatur von 125 °C in den mittleren Bereich der Konvektionszone (33) des Spaltofens (33 plus 41) gefördert. Durch die abziehenden Rauchgase aus der Strahlungszone (41) des Spaltofens wird das flüssige 1,2-Dichlorethan auf 210 °C erwärmt. Im Wärmetauscher (34) erfolgt der Energieausgleich zwischen den an das flüssige 1,2-Dichlorethan Energie abgebenden Bereich (33) und der für die 1,2-Dichlorethan-Verdampfung benötigten Energieaufnahme im ersten Behälter (40). Zu diesem Zweck wird die Standhöhe des flüssigen 1,2-Dichlorethans im zweiten Behälter (35) mit einer üblichen Einrichtung (LIC) gemessen und mit diesem Meßwert als Regelgröße die erforderliche Menge des unter einem Druck von 2,5 MPa stehenden Kesselspeisewassers als Kühlmittel dem Wärmetauscher (34) über die Leitung (54) zugeführt. Für die Abkühlung sind 180 dm³ des Kesselspeisewassers erforderlich, das sich dabei von 100 °C auf 130 °C erwärmt und den Wärmetauscher (34) über die Leitung (55) verläßt. Die rückgewonnene Energiemenge beträgt 73,5 kJ/kg Vinylchlorid.

Das auf etwa 195 °C abgekühlte 1,2-Dichlorethan wird über eine Rohrschleife mit gleichmäßig verteilten Öffnungen (37) dem zweiten Behälter (35) zugeführt und mischt sich dort mit dem heißeren 1,2-Dichlorethan, das vom ersten Behälter (40) durch die Rohre (38) in den zweiten Behälter aufgestiegen ist, wobei ein Teil dieses 1,2-Dichlorethans verdampft.

Das verdampfte 1,2-Dichlorethan wird mit 262 °C frei von flüssigen oder festen Bestandteilen über die Leitung (56) in den unteren Teil der Konvektionszone (33) des Spaltofens eingeführt und dort auf etwa 400 °C überhitzt. Von hier wird es über die Leitung (57) in die Strahlungszone (41) weitergeleitet und auf 525 °C erhitzt.

Während der Überhitzung des Gases auf 525 °C wird ein Teil des 1,2-Dichlorethans in Vinylchlorid und Chlorwasserstoff gespalten. Die heißen Spaltgase werden, wie oben bereits erwähnt, dem ersten Behälter (40) über die Leitung (48) zugeführt und verlassen diesen Behälter mit einer Temperatur von 268 °C. Die durchschnittliche Abkühlgeschwindigkeit der Spaltgase im ersten Behälter (40) beträgt 41,5 °C/s, das sind 1/12,6 der Eingangs-Temperatur (525 °C) pro Sekunde. Über die Leitung (44) werden diese Spaltgase einer weiteren Kühlung nach dem Stande der Technik (nicht in Figur 3 dargestellt) zugeführt, wobei sie teilweise kondensieren. Aus dem durch thermische Spaltung entstandenen Stoffgemisch wird nach bekanntem Verfahren in einer Kolonne (ebenfalls nicht in Figur 3 dargestellt) Chlorwasserstoff bei einer Kopftemperatur von - 24 °C abgetrennt. Der Druck am Kopf dieser Kolonne wird so eingestellt, daß das gasförmige verdampfte 1,2-Dichlorethan mit einer Temperatur von 262 °C den zweiten Behälter (35) verläßt. In diesem Behälter verdampfen 776 kg/h 1,2-Dichlorethan bei einem Druck von 3,5 MPa. Je Quadratmeter Oberfläche, der im zweiten Behälter (35) als ruhend angenommenen Flüssigkeitsoberfläche, werden 2 780 kg/h 1,2-Dichlorethan verdampft. Aus dem Unterteil des ersten Behälters (40) werden 24 kg/h flüssiges 1,2-Dichlorethan abgezogen und über die Leitung (45) einer Kolonne, in der 1,2-Di-chlorethan über Kopf abdestilliert wird (nicht in Figur 3 dargestellt), zugeführt. Das sind 3,0 kg/h aus dem ersten Behälter (40) abgezogenes 1,2-Dichlorethan je 100 kg/h des in den zweiten Behälter (35) frisch eingespeisten 1,2-Dichlorethans. Die mittlere Verweilzeit des 1,2-Dichlorethans, im ersten und zweiten Behälter zusammen, beträgt 48 min.

Die Brenner im Spaltofen werden über die Leitung (43) mit 0,071 Nm³ Brennstoff (Methan) je Kilogramm erzeugtes Vinylchlorid beaufschlagt. Im oberen Abschnitt der Konvektionszone (33) des Spaltofens werden über die Leitung (51) 110 kg/h Kesselspeisewasser mit 100 °C zugeführt und auf 130 °C erwärmt. Die hierbei rückgewonnene Energie beträgt 45,5 kJ/kg Vinylchlorid. Über die Leitung (58) werden 290 dm³/h Heißwasser von 130 °C abgegeben, die hierbei rückgewonnene Energiemenge beträgt 119 kJ/kg Vinylchlorid.

Der Umsatz bei der Spaltung des 1,2-Dichlorethans in der Strahlungszone (41) des Spaltofens (33 plus 41) beträgt 65 %. Es werden 312 kg/h Vinylchlorid produziert.

Nach 9 Monaten Laufzeit ist der Wärmeübergang zwischen den heißen Vinylchlorid enthaltenden Gasen aus dem Spaltofen und dem flüssigen 1,2-Dichlorethan im ersten Behälter (40) nahezu unverändert. Die Temperaturdifferenz zwischen den heißen Gasen aus dem Spaltofen, die über die Leitung (44) aus dem ersten Behälter (40) abgeführt werden und dem gasförmigen 1,2-Dichlorethan, das den zweiten Behälter (35) verläßt und über die Leitung (56) dem Spaltofen zugeführt wird, beträgt 10 °C. Auf den Wärmetauschflächen wird weder auf der Seite der heißen Spaltgase noch auf der Seite des flüssigen 1,2-Dichlorethans ein nennenswerter Belag festgestellt.

Die aus den Rauchgasen des Spaltofens durch Erzeugung von Heißwasser zurückgewonnene Energie beträgt 119 kJ/kg Vinylchlorid, dies entspricht einer Brennstoff-(Methan)-Menge von 0,003 Nm³/kg Vinylchlorid. Damit reduziert sich der effektive Heizgasverbrauch auf 0,068 Nm³/kg Vinylchlorid, das sind nur 66,4 % von der Menge (100 %), die sich aus dem Vergleichsversuch ergibt. Die Energie-Einsparung beträgt demnach 33,6 %, neben einer Vergrößerung des Spaltumsatzes von 55 auf 65 % und einer Verlängerung der Laufzeit des Spaltofens von 6 auf 9 Monate.

**Patentansprüche**

1. Verfahren zur Herstellung von Vinylchlorid durch thermische Abspaltung von Chlorwasserstoff aus 1,2-Dichlorethan in einem Spaltofen, wobei flüssiges 1,2-Dichlorethan mit dem heißen, Vinylchlorid enthaltenden Gas, das den Spaltofen verläßt, indirekt erwärmt, verdampft und das gasförmige 1,2-Dichlorethan in den Spaltofen eingeführt wird, dadurch gekennzeichnet, daß das 1,2-Dichlorethan in einem ersten Behälter mit dem Vinylchlorid enthaltenden Gas bis zum Siedepunkt erwärmt wird und von dort in einen darüberliegenden zweiten Behälter aufsteigt, in dem es ohne weitere Erwärmung unter geringerem Druck als in dem ersten Behälter teilweise verdampft, wobei das verdampfte 1,2-Dichlorethan in den Spaltofen eingespeist und das nicht-verdampfte 1,2-Dichlorethan in den ersten Behälter zurückgeführt wird.

2. Verfahren nach Anspruch 1, dadurch gekennzeichnet, daß in den zweiten Behälter frisches, flüssiges 1,2-Dichlorethan eingespeist wird, dessen Temperatur mit Temperiermitteln über die Standhöhe der Flüssigkeit im zweiten Behälter als Regelgröße eingestellt wird.

3. Verfahren nach Anspruch 2, dadurch gekennzeichnet, daß je Quadratmeter Oberfläche der im zweiten Behälter als ruhend angenommenen Flüssigkeit 1 000 bis 10 000 kg pro Stunde 1,2-Dichlorethan verdampft werden.

4. Verfahren nach einem oder mehreren der Ansprüche 1 bis 3, dadurch gekennzeichnet, daß frisches, flüssiges 1,2-Dichlorethan mit einer Temperatur von 150 bis 220 °C in den zweiten Behälter eingeführt wird, wobei diese Temperatur so gewählt wird, daß sie mindestens 20 °C unter der Temperatur liegt, mit der das 1,2-Dichlorethan den zweiten Behälter gasförmig verläßt.

5. Verfahren nach einem oder mehreren der Ansprüche 1 bis 4, dadurch gekennzeichnet, daß je 100 kg pro Stunde zwischen dem ersten und dem zweiten Behälter umlaufenden 1,2-Dichlorethans 2 bis 20 kg pro Stunde frisches 1,2-Dichlorethan in den zweiten Behälter eingespeist werden.

6. Verfahren nach einem oder mehreren der Ansprüche 1 bis 5, dadurch gekennzeichnet, daß die Strömung des flüssigen, erwärmten 1,2-Dichlorethans vom ersten in den zweiten Behälter und des nichtverdampften 1,2-Dichlorethans vom zweiten zurück in den ersten Behälter ohne Anwendung mechanischer Fördermittel erfolgt.

7. Verfahren nach einem oder mehreren der Ansprüche 1 bis 6, dadurch gekennzeichnet, daß ein Teil des flüssigen 1,2-Dichlorethans aus dem ersten Behälter abgezogen, falls erforderlich,von festen Bestandteilen getrennt und in eine Destillationskolonne eingespeist wird.

8. Verfahren nach Anspruch 7, dadurch gekennzeichnet, daß je 100 kg pro Stunde des in den zweiten Behälter frisch eingespeisten flüssigen 1,2-Dichlorethans 0,5 bis 7 kg pro Stunde flüssiges 1,2-Dichlorethan aus dem ersten Behälter abgezogen und einer Destillationskolonne zugeführt werden.

9. Verfahren nach einem oder mehreren der Ansprüche 1 bis 8, dadurch gekennzeichnet, daß die mittlere Verweilzeit des 1,2-Dichlorethans im ersten und zweiten Behälter zusammen 15 bis 90 Minuten beträgt.

10. Verfahren nach einem oder mehreren der Ansprüche 1 bis 9, wobei das Vinylchlorid enthaltende Gas, das nach dem indirekten Wärmetausch den ersten Behälter verläßt, einer Kolonne zur Abtrennung von Chlorwasserstoff zugeführt wird, deren Kopftemperatur -20 bis -50 °C beträgt, dadurch gekennzeichnet, daß der Druck am Kopf dieser Kolonne so eingestellt wird, daß das verdampfte 1,2-Dichlorethan den zweiten Behälter mit einer Temperatur von 170 bis 280 °C verläßt.

11. Verfahren nach einem oder mehreren der Ansprüche 1 bis 10, dadurch gekennzeichnet, daß das frische, flüssige 1,2-Dichlorethan, bevor es in den zweiten Behälter eingespeist wird, in der Konvektionszone des Spaltofens mit dem Rauchgas, das die den Spaltofen heizenden Brenner erzeugen, erwärmt wird.

12. Verfahren nach einem oder mehreren der Ansprüche 1 bis 10, dadurch gekennzeichnet, daß das frische, flüssige 1,2-Dichlorethan, bevor es in den zweiten Behälter eingespeist wird, mit einem Temperiermedium erwärmt wird, das seinerseits in der Konvektionszone des Spaltofens mit dem Rauchgas, das die den Spaltofen heizenden Brenner erzeugen, erwärmt wird.

13. Verfahren nach einem oder mehreren der Ansprüche 1 bis 12, dadurch gekennzeichnet, daß das heiße, Vinylchlorid enthaltende Gas, das den Spaltofen verläßt, während der indirekten Erwärmung des 1,2-Dichlorethans im ersten Behälter mit einer mittleren Abkühlgeschwindigkeit von mindestens pro Sekunde 1/15 der Temperatur in °C, mit der dieses Gas in die Zone der indirekten Erwärmung des 1,2-Dichlorethans eintritt, bis zum Erreichen einer Temperatur, die mindestens 5 °C über der Verdampfungstemperatur des 1,2-Dichlorethans im zweiten Behälter liegt, abgekühlt wird.

14. Vorrichtung zur Durchführung des Verfahrens nach einem oder mehreren der Ansprüche 1 bis 13, bestehend aus zwei geschlossenen zylindrischen Behältern (1; 2), deren Verhältnis von Länge zu Durchmesser 2 bis 8 beträgt, die miteinander durch Rohre verbunden sind und von denen ein Behälter eine Rohrschlange (3) enthält, dadurch gekennzeichnet, daß beide Behälter (1; 2) mit waagrechter oder gegen die Waagrechte leicht geneigter Zylinderachse weitgehend parallel übereinander in einem Abstand angeordnet sind, der untere Behälter (1) die Rohrschlange (3) enthält, vom obersten Teil des unteren Behälters (1) mindestens ein Steigrohr (4) in den oberen Behälter (2) führt und dort in der oberen Hälfte offen endet, vom unteren Teil des oberen Behälters (2) zum unteren Teil des unteren Behälters (1) mindestens ein Verbindungsrohr (5) führt, der untere Behälter (1) im unteren Teil und der obere Behälter (2) im oberen Teil je eine Öffnung (6; 7) enthält und der obere Behälter (2) eine Flüssigkeits-Standhöhenmessung (8) sowie mindestens eine weitere Öffnung (9), von der ein Rohr in den unteren Teil dieses Behälters führt, aufweist.

15. Vorrichtung nach Anspruch 14, dadurch gekennzeichnet, daß das obere Ende des Steigrohres (4) mit einer Haube (10) so bedeckt ist, daß zwischen dieser und dem Rohrende eine ringförmige Öffnung freibleibt.

16. Vorrichtung nach Anspruch 14 oder 15, dadurch gekennzeichnet, daß der obere Behälter (2) nur eine Öffnung (9) enthält, von der ein Rohr in den unteren Teil dieses Behälters führt, wobei am Ende dieses Rohres eine waagrecht liegende, am Ende geschlossene Rohrschleife (12) angebracht ist, die auf ihrer Länge gleichmäßig verteilt Öffnungen enthält.

**Claims**

1. A process for the production of vinyl chloride through thermal elimination of hydrogen chloride from 1,2-dichloroethane in a cracking furnace, liquid 1,2-dichloroethane being warmed indirectly and evaporated using the hot, vinyl chloride-containing gas leaving the cracking furnace, and the gaseous 1,2-dichloroethane being introduced to the cracking furnace, characterised by warming the 1,2-dichloroethane to boiling in a first container with the vinyl chloride-containing gas and ascending said 1,2-dichloroethane from here into a second superimposed container in which it is partially evaporated, without further warming, under a lower pressure than in the first container, the evaporated 1,2-dichloroethane being fed into the cracking furnace and the non-evaporated 1,2-dichloroethane being fed back to the first container.

2. The process as claimed in claim 1, characterised in that fresh, liquid 1,2-dichloroethane whose temperature is adjusted using temperature-control agents through the level of the liquid in the second container as the regulating variable, is fed into the second container.

3. The process as claimed in claim 2, characterised in that 1,000 to 10,000 kg of 1,2-dichloroethane are evaporated per hour per square meter of the surface area of the liquid, taken as resting, in the second container.

4. The process as claimed in one or more of claims 1 to 3, characterised in that fresh, liquid 1,2-dichloroethane having a temperature of 150 to 220°C is introduced into the second container, this temperature being selected so that it is at least 20°C below the temperature at which the 1,2-dichloroethane in gas form leaves the second container.

5. The process as claimed in one or more of claims 1 to 4, characterised in that 2 to 20 kg of fresh 1,2-dichloroethane are fed per hour into the second container per 100 kg of 1,2-dichloroethane circulating per hour between the first and second containers.

6. The process as claimed in one or more of claims 1 to 5, characterised in that the flow of the liquid, warmed 1,2-dichloroethane from the first container into the second and the flow of the nonevaporated 1,2-dichloroethane from the second container back into the first takes place without using mechanical transport means.

7. The process as claimed in one or more of claims 1 to 6, characterised in that a part of the liquid 1, 2-dichloroethane is drawn off from the first container, separated from solid components if necessary, and fed to a distillation column.

8. The process as claimed in claim 7, characterised in that 0.5 to 7 kg of liquid 1,2-dichloroethane are drawn off per hour from the first container and fed to a distillation column per 100 kg of liquid 1,2-dichloroethane freshly fed per hour into the second container.

9. The process as claimed in one or more of claims 1 to 8, characterised in that the average residence time of the 1,2-dichloroethane in the first and second containers together is 15 to 90 minutes.

10. The process as claimed in one or more of claims 1 to 9, the vinyl chloride-containing gas leaving the first container after the indirect heat exchange being fed to a column for the removal of hydrogen chloride, the head temperature of the column being −20 to −50°C, characterised in that the pressure at the head of this column is adjusted so that the evaporated 1,2-dichloroethane leaves the second container with a temperature of 170 to 380°C.

11. The process as claimed in one or more of claims 1 to 10, characterised in that the fresh, liquid 1,2-dichloroethane, before it is fed to the second container, is warmed in the convection zone of the cracking furnace using the exhaust gas which the burners heating the cracking furnace produce.

12. The process as claimed in one or more of claims 1 to 10, characterised in that the fresh, liquid 1,2-dichloroethane, before it is fed to the second container, is warmed using a temperature-control agent which is itself warmed in the convection zone of the cracking furnace using the exhaust gas which the burners heating the cracking furnace produce.

13. The process as claimed in one or more of claims 1 to 12, characterised in that the hot, vinyl chloride-containing gas leaving the cracking furnace is cooled, during the indirect warming of the 1,2-dichloroethane in the first container, at an average cooling rate per second of at least 1/15 of the temperature, in °C, with which this gas enters the indirect warming zone of the 1,2-dichloroethane, until a temperature which is at least 5°C above the evaporation temperature of the 1,2-dichloroethane in the second container is reached.

14. An apparatus for carrying out the process as claimed in one or more of claims 1 to 13, being composed of two closed cylindrical containers (1; 2) whose length to diameter ratio is 2 to 8, which are connected to one another through pipes and of which one container contains a coiled pipe (3), characterised in that the two containers (1; 2) are arranged substantially parallel above one another at a distance with cylinder axes which are horizontal or slightly inclined to the horizontal, the lower container (1) contains the coiled pipe (3), at least one rising pipe (4) leads from the uppermost part of the lower container (1) into the upper container (2) and ends open in the upper half of the latter, at least one connecting pipe (5) leads from the lower part of the upper container (2) to the lower part of the lower container (1), the lower container (1) in the lower part and the upper container (2) in the upper part each contain an aperture (6; 7), and the upper container (2) has a liquid-level measurement device (8) and at least one further aperture (9) from which a pipe leads into the lower part of this container.

15. The apparatus as claimed in claim 14, characterised in that the upper end of the rising pipe (4) is covered with a hood (10) so that an annular aperture remains free between this hood and the end of the pipe end.

16. The apparatus as claimed in claim 14 or 15, characterised in that the upper container (2) only contains one aperture (9) from which a pipe leads into the lower part of this container, a horizontal pipe loop (13) which is closed at the end and which contains apertures distributed uniformly along its length being attached to the end of this pipe.

**Revendications**

1. Procédé pour la préparation de chlorure de vinyle par dissociation thermique de chlorure d'hydrogène, à partir de dichloro-1,2 éthane dans un four de dissociation, procédé dans lequel du dichloro-1,2 éthane liquide est, avec le gaz chaud contenant du chlorure de vinyle et sortant du four de dissociation, indirectement chauffé puis vaporisé, le dichloro-1,2 éthane gazeux étant introduit dans le four de dissociation, caractérisé en ce que le dichloro-1,2 éthane est chauffé dans un premier réacteur, avec le gaz contenant du chlorure de vinyle, jusqu'au point d'ébullition, et, de là, remonte dans un deuxième réacteur, situé au-dessus, dans lequel, sans chauffage supplémentaire, et sous une pression plus faible que dans le premier réacteur, il subit une vaporisation partielle, le dichloro-1,2 éthane vaporisé étant introduit dans le four de dissociation, le dichloro-1,2 éthane non-vaporisé étant renvoyé dans le premier réacteur.

2. Procédé selon la revendication 1, caractérisé en qu'on introduit dans le deuxième réacteur du dichloro-1,2 éthane liquide frais, dont la température est ajustée, à l'aide d'agents de mise en équilibre de la température, le paramètre de régulation étant le niveau du liquide dans le deuxième réacteur.

3. Procédé selon la revendication 2, caractérisé en ce qu'on vaporise 1000 à 10 000 kg/h de dichloro-1,2 éthane par m²d'aire du liquide supposé au repos dans le deuxième réacteur.

4. Procédé selon l'une ou plusieurs des revendications 1 à 3, caractérisé en ce qu'on introduit dans le deuxième réacteur du dichloro-1,2 éthane liquide, frais, à une température de 150 à 220°C, cette température étant choisie de façon à être d'au moins 20°C inférieure à la température à laquelle le dichloro-1,2 éthane quitte le deuxième réacteur sous forme gazeuse.

5. Procédé selon l'une ou plusieurs des revendications 1 à 4, caractérisé en ce qu'on introduit dans le deuxième réacteur, pour 100 kg/h de dichloro-1,2 éthane en recirculation entre le premier et le deuxième réacteurs, 2 à 20 kg/h de dichloro-1,2 éthane frais.

6. Procédé selon l'une ou plusieurs des revendications 1 à 5, caractérisé en ce que l'écoulement du dichloro-1,2 éthane liquide chauffé entre le premier et le deuxième réacteurs, et celui du dichloro-1,2 éthane non vaporisé, du deuxième réacteur vers le premier, s'effectuent sans utilisation de moyens de transport mécaniques.

7. Procédé selon l'une ou plusieurs des revendications 1 à 6, caractérisé en ce qu'une partie du dichloro-1,2 éthane liquide est soutirée du premier réacteur, si nécessaire débarrassée des constituants solides, et introduite dans une colonne de distillation.

8. Procédé selon la revendication 7, caractérisé en ce qu'on soutire du premier réacteur, pour 100 kg/h du dichloro-1,2 éthane liquide frais introduit dans le deuxième réacteur, 0,5 à 7 kg/h de dichloro-1,2 éthane liquide, que l'on envoie dans une colonne de distillation.

9. Procédé selon l'une ou plusieurs des revendication 1 à 8, caractérisé en ce que le temps de séjour moyen du dichloro-1,2 éthane dans l'ensemble constitué du premier et du deuxième réacteurs est de 15 à 90 minutes.

10. Procédé selon l'une ou plusieurs des revendications 1 à 9, dans lequel le gaz contenant du chlorure de vinyle, lequel quitte le premier réacteur après l'échange de chaleur indirect, est envoyé dans une colonne pour séparer le chlorure d'hydrogène, colonne dont la température de tête est de −20 à −50°C, caractérisé en ce que la pression en tête de cette colonne est ajustée de façon que le dichloro-1,2 éthane vaporisé quitte le deuxième réacteur à une température de 170 à 280°C.

11. Procédé selon l'une ou plusieurs des revendications 1 à 10, caractérisé en ce que le dichloro-1,2 éthane liquide frais est, avant d'être introduit dans le deuxième réacteur, chauffé dans la zone de convection du four de dissociation avec les fumées produites par les brûleurs qui chauffent le four de dissociation.

12. Procédé selon l'une ou plusieurs des revendications 1 à 10, caractérisé en ce que le dichloro-1,2 éthane liquide frais, avant d'être introduit dans le deuxième réacteur, est chauffé avec un fluide de mise en équilibre de température, qui pour sa part est chauffé dans la zone de convection du four de dissociation à l'aide des fumées produites par les brûleurs qui chauffent le four de dissociation.

13. Procédé selon l'une ou plusieurs des revendications 1 à 12, caractérisé en ce que le gaz chaud contenant du chlorure de vinyle, qui sort du four de dissociation, est, pendant le chauffage indirect du dichloro-1,2 éthane dans le premier réacteur, refroidi à une vitesse de refroidissement moyenne par seconde d'au moins 1/15 de la température en °C à laquelle ce gaz pénètre dans la zone du chauffage indirect du dichloro-1,2 éthane, jusqu'à atteindre une température d'au moins 5°C supérieure à la température de vaporisation du dichloro-1,2 éthane, dans le deuxième réacteur.

14. Appareillage pour la mise en Ôuvre du procédé selon l'une ou plusieurs des revendication 1 à 13, constitué de deux réacteurs cylindriques fermés (1; 2), présentant entre leur longueur et leur diamètre un rapport de 2 à 8, qui sont reliés l'un à l'autre par des conduites, l'un des deux réacteurs contenant un serpentin (3), caractérisé en ce que les deux réacteurs sont disposés l'un au-dessus de l'autre à une certaine distance l'un de l'autre, d'une manière pratiquement parallèle l'un par rapport à l'autre, l'axe des cylindres étant horizontal ou faisant un angle faible par rapport à l'horizontale, le réacteur inférieur (1) contient le serpentin (3), au moins une colonne montante (4), partant de la partie supérieure du réacteur inférieur (1), conduit dans le réacteur supérieur (2) et, là, débouche dans la moitié supérieure, au moins un tube de liaison (5) partant de la partie inférieure du réacteur (2) pour arriver dans la partie inférieure du réacteur inférieur (1), le réacteur inférieur (1) et le réacteur supérieur (2) contenant chacun, le premier dans sa partie inférieure et le deuxième dans sa partie supérieure, une ouverture (6; 7), et le réacteur supérieur (2) contient un dispositif de mesure du niveau du liquide (8), ainsi qu'au moins une autre ouverture, d'où part une conduite allant dans la partie inférieure de ce réacteur.

15. Appareillage selon la revendication 14, caractérisé en ce que l'extrémité supérieure de la colonne montante (4) est recouverte d'un chapeau (10) de façon qu'il reste une ouverture annulaire entre ce dernier et l'extrémité de la colonne montante.

16. Appareillage selon la revendication 14 ou 15, caractérisé en ce que le réacteur supérieur (2) ne contient qu'une ouverture (9), de laquelle part un tube allant dans la partie inférieure de ce réacteur, tandis qu'à l'extrémité de ce tube est disposé un serpentin (12), horizontal et fermé en son extrémité, qui contient des ouvertures réparties uniformément sur toute sa longueur.

**Fig.1**

Fig.2

Fig. 3

EP 0 264 065 B1

Fig. 4

EP 0 264 065 B1

Fig. 5